# DEMANDE DE BREVET EUROPEEN

(11) **EP 3 527 264 A1**
(43) Date de publication de la demande: **21.08.2019**
(21) Numéro de dépôt: 18156798.3
(22) Date de dépôt: 14.02.2018
(51) Int. Cl.: A61Q 5/12, A61K 8/92, A61K 8/37, A61K 8/64, A61K 8/41, A61K 8/9711, A61K 8/60, A61Q 5/02

(54) **COMPOSITIONS DE SOIN CAPILLAIRE**

(71) Demandeur: Lam & Lam Partners, 28000 Chartres (FR)
(72) Inventeur: BELLE WANGUE, Régine Béatrice, 92120 MONTROUGE (FR); CRAMER, Morane, 45390 PUISEAUX (FR)
(74) Mandataire: Vialle-Presles, Marie José

(57) **Abrégé**

L'invention est relative à des compositions de soin capillaire combinant du beurre de karité, de la cire de carnauba, des triglycérides capryliques/capriques, un hydrolysat acide de protéines, de la stéaramidopropyl diméthylamine, un extrait d'algues et du xylitylglucoside.

Ces compositions sont utilisables notamment pour le soin et l'entretien des cheveux secs, et en particulier des cheveux afro-métissés, et/ou des cheveux des bébés et des enfants.

## Description

L'invention est relative à des compositions d'hygiène et de soin capillaire, utilisables pour le soin des cheveux des bébés et des enfants et convenant notamment aux cheveux afro-métissés.

Selon la classification la plus habituellement utilisée à l'heure actuelle, on distingue schématiquement 3 types principaux de cheveux : le cheveu asiatique, le cheveu caucasien et le cheveu africain. Ils diffèrent principalement par leur aspect, leur géométrie, leurs propriétés mécaniques et leur teneur en eau (Tanus et al., An Bras Dermatol. 2015; 90(4):450-67). Les principaux critères de classification utilisés sont les suivants :
- Le diamètre de la tige pilaire et la forme de sa section : le cheveu asiatique a le diamètre le plus important, homogène sur toute sa longueur, et la section de la tige est circulaire, résultant en un cheveu raide. Le cheveu africain a une section elliptique, un diamètre plus faible que celui du cheveu asiatique et qui varie en différents endroits de la tige pilaire. La tige pilaire a tendance à s'entortiller sur elle-même, formant de nombreux coudes donnant à la chevelure un aspect crépu. Le cheveu caucasien a un diamètre plus faible que celui du cheveu asiatique et une section circulaire à légèrement ovale. L'aspect de la chevelure varie de raide à légèrement bouclé.
- Les propriétés mécaniques : le cheveu africain est plus fragile et moins résistant à la traction que les cheveux caucasien et asiatique. Ceci s'explique entre autres par la forme elliptique et irrégulière de sa tige pilaire, qui présente au niveau des coudes des zones d'étranglement constituant des points de fragilité.
- La teneur en eau : la teneur en eau du cheveu africain est moindre que celle des cheveux asiatiques et caucasiens. En effet, la capacité d'absorption d'eau et la liaison des molécules d'eau avec les protéines de la tige capillaire dépendent des propriétés physiques et de l'architecture de celle-ci. Notamment, le sébum joue un rôle majeur dans le maintien de l'hydratation du cheveu, en se répandant par capillarité depuis les glandes sébacées situées à la racine du cheveu sur toute la longueur de celui-ci, créant ainsi une gaine protectrice freinant la perte d'eau. Or, dans le cas du cheveu africain, du fait notamment de la structure de la tige capillaire, la répartition du sébum le long de celle-ci se fait mal, ce qui aboutit à un aspect terne et sec des cheveux.

La classification conventionnelle selon les 3 types principaux mentionnés ci-dessus présente l'inconvénient de ne pas refléter suffisamment la grande variété de types de cheveux résultant du métissage, et notamment la diversité des frisures.

Plus récemment il a été proposé un système de classification indépendant de l'origine ethnique, basé sur les différents degrés de frisure des cheveux (Loussouarn et al., International Journal of Dermatology, 46, (Suppl.1), 2-6, 2007).

Ce système s'appuie sur le diamètre de la courbure des boucles, l'index de bouclage, le nombre d'ondulations, et le nombre de torsades sur les cheveux. Il distingue ainsi 8 types de cheveux selon leur degré de frisure : Type I (raide), Type II (large ondulation), Type III (ondulé), Type IV (bouclé), Type V (très bouclé), Type VI (enroulé), Type VII (très enroulé), Type VIII (très enroulé et torsadé).

Dans le cadre de l'exposé de la présente invention, on désigne par cheveux « afro-métissés » les cheveux des Types IV à VIII de cette classification.

Il a été observé que plus le cheveu est frisé, plus sa résistance au stress mécanique est faible, et plus le risque de cassure est important. Ceci peut être lié notamment aux zones de fragilité au niveau des boucles, ainsi qu'à l'accentuation de la sècheresse de la fibre capillaire consécutive à la difficulté de lubrification par le sébum sur toute sa longueur. En outre, plus le degré de frisure est important, plus la probabilité de formation de noeuds le long des tiges pilaires et entre celles-ci augmente, ce qui accroit les difficultés de démêlage et de coiffage de la chevelure. Les agressions mécaniques répétées exercées lors du coiffage fragilisent encore la fibre capillaire, augmentant d'autant le risque de cassure.

Ces problèmes sont encore accentués dans le cas des enfants, dont les cheveux sont plus fins et plus fragiles que ceux des adultes, et qui produisent moins de sébum protecteur.

La présente invention a pour but de remédier à ces problèmes de fragilité des cheveux afro-métissés, en fournissant des compositions capillaires assurant une gaine protectrice permettant de pallier l'absence ou l'insuffisance de la gaine naturelle de sébum, et permettant en outre d'hydrater et de maintenir l'hydratation de la tige capillaire, et de faciliter le coiffage par une action anti-noeuds.

La présente invention a en premier lieu pour objet une composition de soin capillaire, caractérisée en ce qu'elle comprend la combinaison des constituants suivants :
- du beurre de karité (*Butyrospermum Parkii*) ;
- de la cire de carnauba (*Copernicia Cerifera*) ;
- des triglycérides capryliques/capriques ;
- un hydrolysat acide de protéines ;
- de la stéaramidopropyl diméthylamine ;
- un extrait d'algues ;
- du xylitylglucoside.

Outre les constituants essentiels mentionnés ci-dessus, la composition conforme à l'invention peut contenir des constituants secondaires, qui peuvent être par exemple des adjuvants permettant de faciliter la solubilisation des constituants principaux et/ou de favoriser ou de stabiliser leur mélange, des conservateurs tels qu'anti-microbiens ou antioxydants, des substances tampon, ou bien des composés corrélés à la méthode de synthèse d'un des constituants principaux, tels que des résidus des produits de départ, ou des sous-produits de synthèse.

Lorsque dans l'exposé de la présente invention, des pourcentages sont indiqués, il s'agit de pourcentage en poids, sauf précision contraire.

Habituellement, la composition conforme à l'invention comprend une phase aqueuse représentant au moins 10%, de préférence au moins 20%, et de préférence au moins 30% de la composition. De préférence cette phase aqueuse représente de 30 à 70 % de la composition.

Avantageusement, la cire de carnauba et les triglycérides capryliques/capriques sont sous forme d'une dispersion dans l'eau. Le diamètre particulaire moyen de cette dispersion est de préférence inférieur à 1µm, avantageusement inférieur à 500 nm, et généralement compris entre 100 et 500 nm. Cette dispersion peut contenir de 10 à 25 % de cire de carnauba, et de 25 à 50 % de triglycérides capryliques/capriques. Elle peut contenir en outre de 1 à 5 % d'adjuvants solvants et/ou stabilisateurs tels que notamment le décyl glucoside ou le pentylène glycol.

De préférence, l'hydrolysat acide de protéines est un hydrolysat de protéines végétales, par exemple un hydrolysat de protéines de blé ou de riz, et notamment un hydrolysat de protéines de riz. Avantageusement, ledit hydrolysat acide de protéines est utilisé sous forme d'un complexe ionique avec la stéaramidopropyl diméthylamine, ledit complexe étant de préférence en solution dans un mélange de glycérine et d'eau.

L'extrait d'algues est en particulier un extrait d'algues brunes, de préférence de l'ordre des Laminariales, avantageusement de la famille des Alariacées, en particulier du genre Alaria, et notamment de l'espèce *Alaria esculenta.* Il s'agit de préférence d'un extrait hydroglycolique, les proportions respectives d'eau et de glycol étant généralement de 50 :50. Le glycol peut être par exemple le butylène glycol. Ledit extrait hydroglycolique a généralement une teneur en extrait sec pouvant varier de 0,5 à 3%.

Le xylitylglucoside est généralement disponible sous forme d'un mélange avec les produits de départ à partir desquels il est synthétisé, à savoir le glucose et le xylitol, et avec un sous-produit de sa synthèse, l'anhydroxylitol.

Selon un mode de réalisation préféré d'une composition conforme à l'invention, elle comprend de 5 à 30 %, de préférence de 10 à 20 % de beurre de karité, de 5 à 30 %, de préférence de 10 à 20 % de triglycérides capryliques/capriques, de 5 à 20 %, de préférence de 8 à 15 % de cire de carnauba, de 2 à 15 %, de préférence de 5 à 10 % d'hydrolysat acide de protéines, de 1 à 5 %, de préférence de 1,5 à 4 % de stéaramidopropyl diméthylamine, de 0,1 à 0,5 %, de préférence de 0,15 à 0,4 % (en extrait sec) d'extrait d'algues, et de 1 à 5 %, de préférence de 1,5 à 4 % de xylitylglucoside.

Une composition conforme à l'invention peut également comprendre de 2 à 15 %, de préférence de 4 à 10 % de glycérine, de 2 à 10 %, de préférence de 3 à 8 % de butylène glycol, de 0,5 à 5 %, de préférence de 1 à 3 % de décyl glucoside, de de 0,5 à 5 %, de préférence de 1 à 3 % de pentylène glycol, de 0,5 à 4 %, de préférence de 1 à 3 % d'anhydroxylitol, de 0,1 à 1,5 %, de préférence de 0,5 à 1 % de xylitol, et de 0,1 à 0,5 %, de préférence de 0,2 à 0,4 % de glucose. Elle peut en outre éventuellement comprendre de 0,5 à 3 %, de préférence de 1 à 2 % de tocophérol en tant qu'anti-oxydant, et de l'acide citrique en quantité utile pour conférer un pH de 5 à 5,5

Les compositions de soin capillaire conformes à l'invention peuvent être utilisées telles quelles, ou incorporées dans tout type de formulation capillaire. Il peut s'agir notamment de shampooings, d'après-shampooings avec ou sans rinçage, de masques ou de crèmes capillaires, de produits de coiffage destinés à faciliter le démêlage et/ou la mise en forme de la coiffures, etc. ,

Les formulations capillaires dans lesquelles peuvent être incorporées les compositions conformes à l'invention comportent également des ingrédients supplémentaires, généralement des excipients et le cas échéant des principes actifs. Ces ingrédients supplémentaires peuvent être choisis par l'homme du métier, parmi ceux habituellement mis en oeuvre dans ce type de formulations, en fonction notamment de l'usage à laquelle est destinée la formulation, et du public à laquelle elle s'adresse. De la même manière, la proportion dans laquelle la composition conforme à l'invention est incorporée dans la formulation finale peut être choisie par l'homme du métier, en fonction des mêmes critères.

Les formulations capillaires comprenant une composition conforme à l'invention font également partie de l'objet de la présente invention.

La présente invention a également pour objet l'utilisation d'une composition de soin capillaire ou d'une formulation capillaire conforme à l'invention pour le soin et l'entretien des cheveux secs, et des cheveux des bébés et des enfants. Elles permettent d'améliorer l'aspect, de faciliter le coiffage, et de diminuer le risque de cassure de ces cheveux fragiles. En particulier, les compositions de soin capillaire sont avantageusement utilisables pour le soin et l'entretien des cheveux afro-métissés.

La présente invention est illustrée par le complément de description qui suit, qui se réfère à des exemples non limitatifs de préparation d'une composition de soin capillaire conforme à l'invention et de formulations incorporant ladite composition.

### EXEMPLE 1 : COMPOSITION DE SOIN CAPILLAIRE

Cette composition est obtenue par mélange des constituants listés dans le Tableau I ci-dessous.

**TABLEAU I**

| **NUMERO CAS** | **CONSTITUANT (DENOMINATION INCI)** | **%** (**p/p**) |
|---|---|---|
| 7732-18-5 | AQUA (WATER) | 37,5000 |
| 194043-92-0 | BUTYROSPERMUM PARKII BUTTER | 13,9999 |
| 73398-61-5 | CAPRYLIC/CAPRIC TRIGLYCERIDE | 13,0000 |
| 8015-86-9 | COPERNICIA CERIFERA WAX | 9,0000 |
| 156715-40-1 | HYDROLYZED RICE PROTEIN | 5,6000 |
| 56-81-5 | GLYCERIN | 5,2000 |
| 107-88-0 | BUTYLENE GLYCOL | 4,9000 |
| 7651-02-7 | STEARAMIDOPROPYL DIMETHYLAMINE | 2,4000 |
| - | XYLITYLGLUCOSIDE | 2,0000 |
| 58846-77-8 | DECYL GLUCOSIDE | 1,5000 |
| 5343-92-0 | PENTYLENE GLYCOL | 1,5000 |
| 53448-53-6 | ANHYDROXYLITOL | 1,4000 |
| 10191-41-0 | TOCOPHEROL | 1,0000 |
| 87-99-0 | XYLITOL | 0,6000 |
| 92128-82-0 | ALGAE EXTRACT | 0,2000 |
| 50-99-7 | GLUCOSE | 0,2000 |
| 77-92-9 | CITRIC ACID | 0,0001 |

### EXEMPLE 2 : FORMULATION D'UN SHAMPOOING

La composition de l'exemple 1 est ajoutée, à raison de 1% en poids, aux ingrédients d'une base pour shampooing.

La composition finale du shampooing ainsi obtenu est indiquée dans le Tableau II ci-dessous.

**TABLEAU II**

| **NUMERO CAS** | **CONSTITUANT (DENOMINATION INCI)** | **% (p/p)** |
|---|---|---|
| 7732-18-5 | AQUA (WATER) | 85,077500 |
| 58846-77-8 | DECYL GLUCOSIDE | 4,399000 |
| | SODIUM COCOYL APPLE AMINO ACIDS | 2,040000 |
| 107-41-5 | HEXYLENE GLYCOL | 1,025000 |
| | XYLITYLGLUCOSIDE | 0,882500 |
| 100-51-6 | BENZYL ALCOHOL | 0,870000 |
| 9000-01-05 | ACACIA SENEGAL GUM | 0,825000 |
| 56-81-5 | GLYCERIN | 0,712000 |
| 11138-66-2 | XANTHAN GUM | 0,675000 |
| 53448-53-6 | ANHYDROXYLITOL | 0,574000 |
| 87-99-0 | XYLITOL | 0,546000 |
| 14246-53-8 | CAPRYLOYL GLYCINE | 0,500000 |
| | PARFUM | 0,400000 |
| 156715-40-1 | HYDROLYZED RICE PROTEIN | 0,336000 |
| 585-86-4 | LACTITOL | 0,300000 |
| 7651-02-7 | STEARAMIDOPROPYL DIMETHYLAMINE | 0,144000 |
| 194043-92-0 | BUTYROSPERMUM PARKII BUTTER | 0,139999 |
| 73398-61-5 | CAPRYLIC/CAPRIC TRIGLYCERIDE | 0,130000 |
| 50-99-7 | GLUCOSE | 0,098000 |
| 8015-86-9 | COPERNICIA CERIFERA WAX | 0,090000 |
| 520-45-6 | DEHYDROACETIC ACID | 0,080000 |
| 107-88-0 | BUTYLENE GLYCOL | 0,049000 |
| 112-30-1 | DECYL ALCOHOL | 0,040000 |
| 51981-21-6 | TETRASODIUM GLUTAMATE DIACETATE | 0,040000 |
| 5343-92-0 | PENTYLENE GLYCOL | 0,015000 |
| 10191-41-0 | TOCOPHEROL | 0,010000 |
| 92128-82-0 | ALGAE EXTRACT | 0,002000 |
| 77-92-9 | CITRIC ACID | 0,000001 |

### EXEMPLE 3 : FORMULATION D'UNE EAU LACTEE DEMELANTE

La composition de l'exemple 1 est ajoutée, à raison de 10% en poids, aux ingrédients d'une base pour eau démêlante.

La composition finale de l'eau lactée démêlante ainsi obtenue est indiquée dans le Tableau III ci-dessous.

**TABLEAU III**

| **NUMERO CAS** | **CONSTITUANT (DENOMINATION INCI)** | **% (p/p)** |
|---|---|---|
| 7732-18-5 | AQUA (WATER) | 89,68250 |
| 194043-92-0 | BUTYROSPERMUM PARKII BUTTER | 1,39999 |
| 73398-61-5 | CAPRYLIC/CAPRIC TRIGLYCERIDE | 1,30000 |
| 56-81-5 | GLYCERIN | 0,92000 |
| 8015-86-9 | COPERNICIA CERIFERA WAX | 0,90000 |
| 100-51-6 | BENZYL ALCOHOL | 0,87000 |
| 107-41-5 | HEXYLENE GLYCOL | 0,77500 |
| - | SODIUM COCOYL APPLE AMINO ACIDS | 0,76500 |
| 156715-40-1 | HYDROLYZED RICE PROTEIN | 0,56000 |
| 14246-53-8 | CAPRYLOYL GLYCINE | 0,50000 |
| 107-88-0 | BUTYLENE GLYCOL | 0,49000 |
| 87-99-0 | XYLITOL | 0,36000 |
| 585-86-4 | LACTITOL | 0,30000 |
| | XYLITYLGLUCOSIDE | 0,26250 |
| 7651-02-7 | STEARAMIDOPROPYL DIMETHYLAMINE | 0,24000 |
| 58846-77-8 | DECYL GLUCOSIDE | 0,15000 |
| 5343-92-0 | PENTYLENE GLYCOL | 0,15000 |
| 53448-53-6 | ANHYDROXYLITOL | 0,14000 |
| 10191-41-0 | TOCOPHEROL | 0,10000 |
| 520-45-6 | DEHYDROACETIC ACID | 0,08000 |
| 92128-82-0 | ALGAE EXTRACT | 0,02000 |
| 50-99-7 | GLUCOSE | 0,02000 |
| 51981-21-6 | TETRASODIUM GLUTAMATE DIACETATE | 0,01500 |
| 77-92-9 | CITRIC ACID | 0,00001 |

### EXEMPLE 4 : FORMULATION D'UN BAUME HYDRATANT

La composition de l'exemple 1 est ajoutée, à raison de 10% en poids, aux ingrédients d'une base pour baume hydratant.

La composition finale du baume hydratant ainsi obtenu est indiquée dans le Tableau IV ci-dessous.

**TABLEAU IV**

| **NUMERO CAS** | **CONSTITUANT (DENOMINATION INCI)** | % **(p/p)** |
|---|---|---|
| 7732-18-5 | AQUA (WATER) | 80,6965 0 |
| 232-428-0 | PERSEA GRATISSIMA OIL | 6,00000 |
| 111286-86-3 | HYDROXYETHYL ACRYLATE/SODIUM ACRYLOYLDIMETHYL TAURATE COPOLYMER | 1,70000 |
| 5333-42-6 | OCTYLDODECANOL | 1,49400 |
| 194043-92-0 | BUTYROSPERMUM PARKII BUTTER | 1,39999 |
| 73398-61-5 | CAPRYLIC/CAPRIC TRIGLYCERIDE | 1,30000 |
| 56-81-5 | GLYCERIN | 0,92000 |
| 8015-86-9 | COPERNICIA CERIFERA WAX | 0,90000 |
| 100-51-6 | BENZYL ALCOHOL | 0,87000 |
| 107-41-5 | HEXYLENE GLYCOL | 0,62500 |
| 156715-40-1 | HYDROLYZED RICE PROTEIN | 0,56000 |
| 14246-53-8 | CAPRYLOYL GLYCINE | 0,50000 |
| 423772-95-6 | OCTYLDODECYL XYLOSIDE | 0,50000 |
| 107-88-0 | BUTYLENE GLYCOL | 0,49000 |
| 87-99-0 | XYLITOL | 0,36000 |
| 585-86-4 | LACTITOL | 0,30000 |
| | XYLITYLGLUCOSIDE | 0,26250 |
| 7651-02-7 | STEARAMIDOPROPYL DIMETHYLAMINE | 0,24000 |
| 58846-77-8 | DECYL GLUCOSIDE | 0,15000 |
| 5343-92-0 | PENTYLENE GLYCOL | 0,15000 |
| 53448-53-6 | ANHYDROXYLITOL | 0,14000 |
| 9005-67-8 | POLYSORBATE 60 | 0,11000 |
| 71902-01-7 | SORBITAN ISOSTEARATE | 0,11000 |
| 10191-41-0 | TOCOPHEROL | 0,10000 |
| 520-45-6 | DEHYDROACETIC ACID | 0,08000 |
| 92128-82-0 | ALGAE EXTRACT | 0,02000 |
| 50-99-7 | GLUCOSE | 0,02000 |
| 58-86-6 | XYLOSE | 0,00200 |
| 77-92-9 | CITRIC ACID | 0,00001 |

## Revendications

1. Composition de soin capillaire, **caractérisée en ce qu'**elle comprend la combinaison des constituants suivants :
- du beurre de karité ;
- de la cire de carnauba ;
- des triglycérides capryliques/capriques ;
- un hydrolysat acide de protéines ;
- de la stéaramidopropyl diméthylamine ;
- un extrait d'algues ;
- du xylitylglucoside.

2. Composition de soin capillaire selon la revendication 1, **caractérisée en ce qu'**elle comprend en outre de la glycérine, du butylène glycol, du décyl glucoside, du pentylène glycol, de l'anhydroxylitol, du xylitol, et du glucose.

3. Composition de soin capillaire selon une quelconque des revendications 1 ou 2, **caractérisée en ce qu'**elle comprend de 5 à 30 % de beurre de karité, de 5 à 30 % de triglycérides capryliques/capriques, de 5 à 20 % de cire de carnauba, de 2 à 15 % d'hydrolysat acide de protéines, de 1 à 5 % de stéaramidopropyl diméthylamine, de 0,1 à 0,5 % d'extrait sec d'algues, et de 1 à 5 % de xylitylglucoside.

4. Composition de soin capillaire selon une quelconque des revendications 2 ou 3, **caractérisée en ce qu'**elle comprend de 2 à 15 % de glycérine, de 2 à 10 % de butylène glycol, de 0,5 à 5 % de décyl glucoside, de 0,5 à 5 %, de pentylène glycol, de 0,5 à 4 % d'anhydroxylitol, de 0,1 à 1,5 % de xylitol, et de 0,1 à 0,5 % de glucose.

5. Formulation capillaire comprenant une composition de soin capillaire selon une quelconque des revendications 1 à 4.

6. Utilisation d'une composition de soin capillaire selon une quelconque des revendications 1 à 4, ou d'une formulation capillaire selon la revendication 5 pour le soin et l'entretien des cheveux secs.

7. Utilisation d'une composition de soin capillaire selon une quelconque des revendications 1 à 4, ou d'une formulation capillaire selon la revendication 5 pour le soin et l'entretien des cheveux d'un bébé ou d'un enfant.

8. Utilisation selon une quelconque des revendications 6 ou 7, **caractérisée en ce que** lesdits cheveux sont des cheveux afro-métissés.
